# FASCICULE DE BREVET EUROPEEN

(11) **EP 0 947 175 B1**
(45) Date de publication et mention de la délivrance du brevet: **21.05.2003**
(21) Numéro de dépôt: 99400599.9
(22) Date de dépôt: 11.03.1999
(51) Int. Cl.: A61B 17/70

(54) **Dispositif d'ostéosynthése rachidienne adaptable aux différences d'alignement, d'angulation et d'enfoncement des vis pédiculaires**
Vorrichtung zur Wirbelsäulenosteosynthese, welche an der Stellung eines Pedikelschraube anpassbar ist
Spinal osteosynthesis device adaptable to the position of a pedicle screw

(30) Priorité: 03.04.1998 FR 9804187
(43) Date de publication de la demande: 06.10.1999
(73) Titulaire: EUROSURGICAL, 62217 Beaurains (FR)
(72) Inventeur: Viart, Guy, 62128 Saint Leger (FR)
(74) Mandataire: Schmitt, John

(56) Documents cités:
- US-A- 5 312 404
- US-A- 5 476 463
- US-A- 5 628 740

## Description

La présente invention a pour objet un dispositif d'ostéosynthèse rachidienne, comprenant au moins une tige vertébrale, des vis pédiculaires et des connecteurs de liaison entre la tige et les vis, comprenant deux parties respectivement traversées par la vis et par la tige.

Un problème soulevé par la mise en place de telles instrumentations rachidiennes consiste à les adapter aux différences d'alignement, d'angulation et d'enfoncement des vis pédiculaires. La plupart des instrumentations rachidiennes de ce type actuellement connues sont en fait très difficilement adaptables à ces différences d'implantation. Lorsqu'elles le sont, elles ne le sont que partiellement, et par conséquent n'intègrent pas toutes les variations de ces paramètres. Les instrumentations existantes sont en outre généralement complexes en raison de leur nombre élevé de pièces et exigent des chirurgiens, pour les adapter à des désalignements des vis pédiculaires, de choisir des connecteurs appropriés. En outre, si les vis pédiculaires présentent des différences de profondeur d'ancrage d'une vis à l'autre, l'adaptation à celles-ci est difficile ; le chirurgien effectue le serrage de vis qui risquent alors de s'arracher. En définitive, le blocage de certaines vis sur leurs connecteurs n'est parfois pas satisfaisant en raison de ces désalignements et différences de profondeur d'ancrage, que les mesures compliquées effectuées par le chirurgien ne parviennent pas toujours à compenser.

La mise en place des instrumentations existantes est également rendue délicate par la technique de la visée pédiculaire : suivant celle-ci le chirurgien tire une ligne fictive reliant les différents points d'implantation théoriques des vis reliant les pédicules, puis vise dans chacun des secteurs les emplacements des vis. En fait certains chirurgiens non spécialisés dans la chirurgie du rachis choisissent de viser au milieu de chaque secteur, ce qui ne correspond pas nécessairement au meilleur alignement des vis.

Par ailleurs on connaît des vis pédiculaires dites « polyaxiales » qui permettent une adaptation aux différences d'angulation des vis pédiculaires, mais difficilement en alignement et plus encore en profondeur.

On connaît aussi une instrumentation rachidienne avec des connecteurs qui présentent une lumière oblongue, s'étendant transversalement à la tige pour la réception des vis pédiculaires. Ces connecteurs permettent l'adaptation des vis en alignement, mais non en angulation et en profondeur sans utilisation de rondelles spéciales. Un tel dispositif nécessite la mesure, la sélection et le positionnement de rondelles, opérations généralement délicates. En outre la multiplicité d'éléments intercalés accroît le risque de faillite de la fixation. US-A-5 628 740 montre un dispositif selon le préambule de la revendication 1 de l'invention. Les matériaux utilisés sont rigides. Les connecteurs sont faconnés avec une fente pour obtenir une certaine compressibilité.

L'invention a pour but de proposer un dispositif du type mentionné ci-dessus, comprenant des connecteurs vis-tige permettant une adaptabilité automatique et aisée aux différences de positionnement des vis pédiculaires, aussi bien en alignement, en angulation qu'en enfoncement.

Conformément à l'invention, les connecteurs présentent une lumière oblongue s'étendant transversalement à la tige et adaptée pour être traversée par la vis associée, et au moins un connecteur est réalisé en un matériau malléable, plastiquement déformable dans sa partie traversée par la vis sous l'effet du serrage d'un écrou sur la vis lors de sa mise en place, et indéformable sous l'effet de moments de flexion transmis par le rachis in vivo et dont les valeurs sont inférieures à 4,5 N.m environ.

Le matériau choisi présente donc une grande capacité plastique, correspondant à une capacité d'allongement d'au moins 20% environ à partir d'une contrainte de 275 MPa. Ceci permet par simple serrage des écrous de fixation des vis pédiculaires sur les connecteurs, de créer la déformation plastique nécessaire à l'adaptation des connecteurs aux différentes vis pédiculaires et à la tige qui les relie. Ainsi le geste opératoire de mise en place par le chirurgien du matériel d'ostéosynthèse dans les arthrodèses du rachis est considérablement facilité.

A titre d'exemple non limitatif, le connecteur peut être en titane pur par exemple selon la nuance ISO 5832-2, qui devient malléable et plastiquement déformable à partir d'un effort de 53 daN exercé par l'outil de serrage de l'écrou de fixation de la vis au connecteur correspondant.

Suivant un mode de réalisation préféré du dispositif visé par l'invention, seuls les connecteurs intermédiaires sont réalisés en un matériau déformable plastiquement, tandis que les connecteurs d'extrémité sont indéformables aux efforts qu'ils subissent lors de leur mise en place et ultérieurement par les mouvements du patient.

D'autres particularités et avantages de l'invention apparaîtront au cours de la description qui va suivre, faite en référence aux dessins annexés qui en illustrent une forme de réalisation à titre d'exemple non limitatifs.

La figure 1 est une vue de dessus en plan, à échelle agrandie, d'une forme de réalisation du connecteur du dispositif d'ostéosynthèse rachidienne visé par l'invention.

La figure 2 est une vue en perspective partielle, à échelle agrandie, d'un dispositif d'ostéosynthèse rachidienne équipé d'un connecteur conforme à la figure 1 qui s'est adapté par déformation plastique pour relier efficacement la tige à la vis pédiculaire.

La figure 3 est une vue en perspective partielle sous un autre angle, du dispositif de la figure 2.

La figure 4 est une vue en élévation latérale à échelle réduite, d'un dispositif d'ostéosynthèse rachidienne selon l'invention, mis en place sur un segment rachidien correspondant.

La figure 5 est une vue en élévation postérieure du dispositif de la figure 4.

La figure 6 est une vue en élévation latérale partielle d'un dispositif selon les figures 2 et 3 au début de la phase de serrage de l'écrou sur la vis et le connecteur.

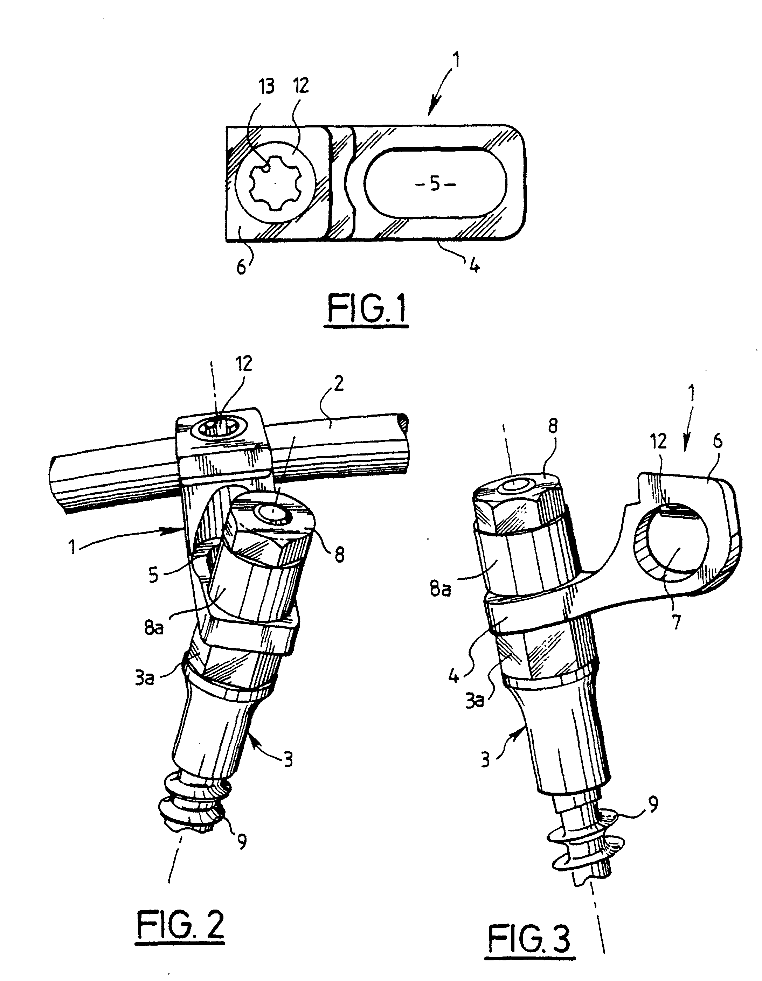

On voit aux figures 1 à 3 un connecteur 1 de liaison entre une tige vertébrale 2 et une vis pédiculaire 3 d'ancrage osseux d'un dispositif d'ostéosynthèse rachidienne.

Le connecteur 1 présente un profil sensiblement en L, dans la grande branche 4 duquel est agencée une lumière oblongue 5 adaptée pour pouvoir être traversée par la vis associée 3, avec une liberté de positionnement dans cette lumière 5 dans la direction transversale à la tige 2.

Dans la petite branche 6 du connecteur 1 est agencé un trou 7 de passage de la tige 2. La vis pédiculaire 3 peut être bloquée dans la position voulue sur le connecteur 1 au moyen d'un écrou 8 à l'embase cylindrique 8a, vissé sur l'extrémité filetée de la vis 3 faisant saillie du connecteur 1. La branche 4 du connecteur 1 est intercalée entre la tête hexagonale 3a et l'embase cylindrique 8a de l'écrou 8 enfilé sur la tige. La tige 2 peut être bloquée dans la branche 6 au moyen d'une vis 12 présentant une empreinte intérieure 13 pour un outil de vissage et qui vient se visser dans un trou taraudé de la branche 6.

Le connecteur 1 est réalisé en un matériau malléable, plastiquement déformable, ayant une capacité d'allongement d'au moins 20 % environ. Le matériau est choisi pour pouvoir être plastiquement déformable à partir d'un effort F de 53 daN, ce qui correspond à un moment de flexion de 4,5N.m exercé sur la vis d'ancrage osseux 3.

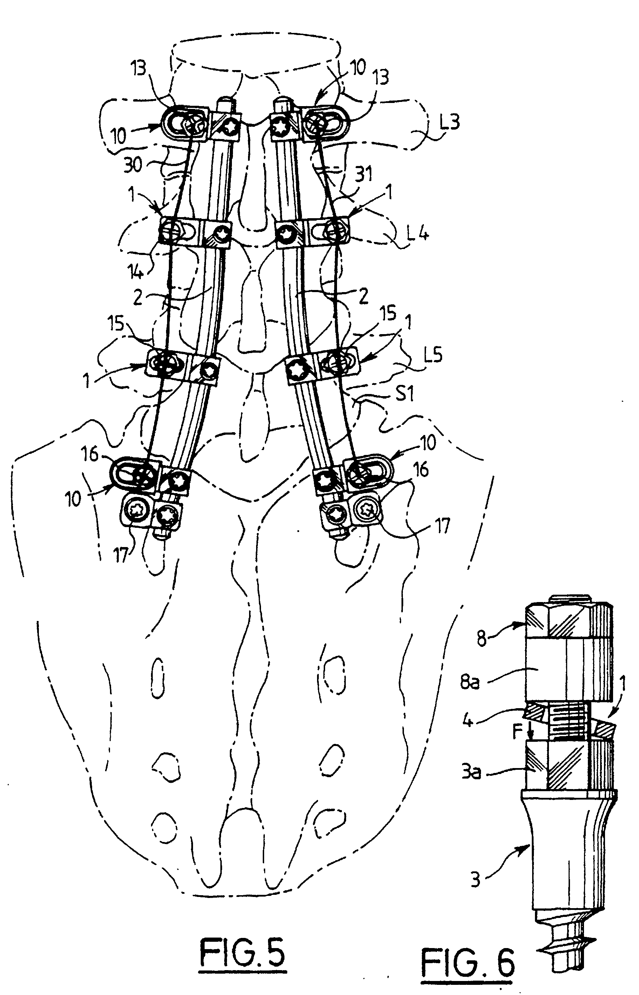

Un tel matériau à grande capacité plastique permet par simple serrage des écrous 8 de fixation des vis 3 aux attaches constituées par des connecteurs 1, de créer dans les branches 4 traversées par les vis 3, par l'effort F de flexion exercé sur ces branches 4 la déformation plastique nécessaire à l'adaptation des différentes vis pédiculaires et de la tige 2 qui les relie, à l'anatomie particulière du segment rachidien considéré. En effet cette possibilité de déformation plastique dans la gamme des efforts F de serrage et de torsion (Fig.6) exercés par le chirurgien sur le connecteur 1 permet de déformer celui-ci de manière appropriée, par exemple comme visible aux figures 2 et 3, pour l'adapter aux différences d'alignement, d'angulation et d'enfoncement des vis d'ancrage osseux 3. La branche 4 du connecteur 1 est plastiquement déformable par serrage de l'écrou 8 sur la vis 3 lors de sa mise en place, et indéformable sous l'effet des moments de flexion transmis par le rachis in vivo dont les valeurs sont inférieures à 4,5N.m environ.

Le titane pur, selon la nuance ISO 5832-2, constitutif du connecteur 1 ne présente pas après déformation le phénomène de surécrouissage, donc d'amorce de rupture. Ce matériau est très ductile et apte à subir des torsions.

Un second matériau possible est l'acier inoxydable 316L implantable « recuit », qui a des caractéristiques de ductilité élevées.

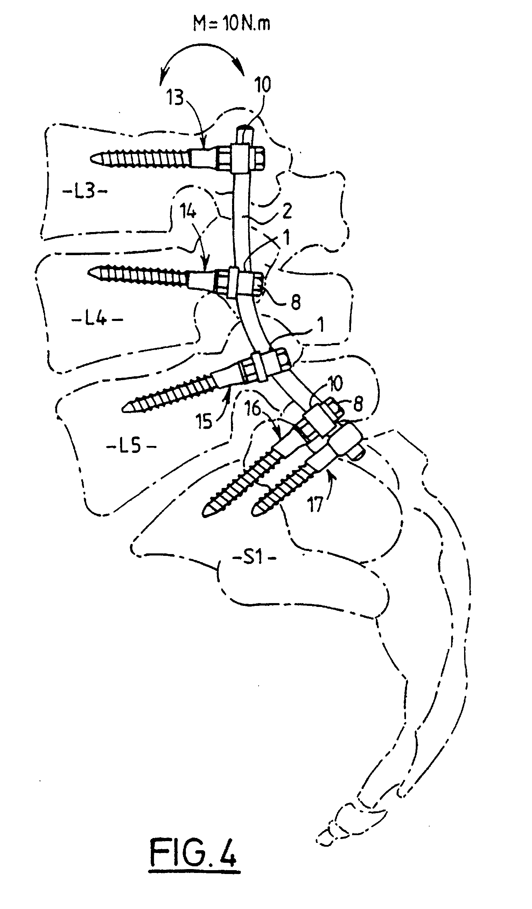

La conception de ce connecteur 1 a été rendue possible par l'observation et l'analyse biomécanique des fixations pédiculaires multisegmentaires dans le traitement des pathologies rachidiennes. Le comportement biomécanique du rachis instrumenté par fixation multisegmentaire à vis pédiculaires a été analysé sur un modèle mathématique tridimensionnel par éléments finis. Cette analyse a permis de mettre en évidence des différences importantes au niveau de chaque vertèbre en matière de sollicitations. En effet, cette analyse biomécanique, effectuée sur une instrumentation rachidienne telle que celle des figures 4 et 5, a permis de mesurer les sollicitations subies par les éléments d'ancrage osseux aux différents niveaux du segment.

L'instrumentation des figures 4 et 5 comprend deux tiges vertébrales 2 lombo-sacrées, s'étendant sur les trois vertèbres lombaires L3, L4, L5 et le sacrum S1. A chaque tige 2 sont associées trois vis pédiculaires 13, 14, 15 ancrées respectivement dans les vertèbres lombaires L3, L4, L5, tandis que deux vis 16, 17, sont ancrées dans le sacrum S1. Ces différentes vis 13...17 subissent des variations d'alignement correspondant aux tracés 30, 31 reliant les vis respectives de chaque tige 2. L'analyse biomécanique d'un chargement du rachis en flexion à 10N.m a permis de mesurer les moments de flexion M exercés sur les vis respectives : en L3 ce moment M a été de 1 N.m, en L4 de 0,08N.m, en L5 de 0,3N.m, et en S1 de 0,6N.m.

Il apparaît donc que les sollicitations des ancrages intermédiaires 14, 15 sont très nettement inférieures à celles des extrémités 13, 16, 17. Il en découle que les vis pédiculaires intermédiaires 14, 15 ainsi que leurs systèmes de connexion 1 avec la tige 2 sur les vertèbres intermédiaires L4, L5 sont peu sollicités.

C'est ainsi que le connecteur 1 selon l'invention a été développé en tenant compte de ces faibles sollicitations pour les ancrages intermédiaires, afin de proposer aux chirurgiens de nouvelles possibilités vers une plus grande versatilité et une simplicité accrue dans la mise en place des fixations d'arthrodèses rachidiennes. En effet le connecteur 1 malléable et déformable plastiquement prévu par l'invention présente la particularité d'être adaptable à un quelconque positionnement des vis pédiculaires, tout en permettant une fixation efficace sans dégradation de l'ancrage osseux.

Les connecteurs 1 selon l'invention sont utilisés uniquement pour les ancrages intermédiaires d'une instrumentation, par exemple les vis 14, 15 sur le montage des figures 4 et 5. Par contre les ancrages d'extrémité (vis 13, 16, 17) sont associés à des connecteurs 10 ayant la même structure que les connecteurs 1 notamment la lumière oblongue 5, mais indéformables dans la gamme des efforts de flexion et de serrage exercés par le chirurgien et des efforts de flexion subis par ces éléments d'ancrage une fois mis en place sur le patient, c'est-à-dire supérieurs à 8 N.m. Les connecteurs 10 peuvent être par exemple en alliage de titane.

Les connecteurs intermédiaires 1 s'adaptent plastiquement aux différences d'angulation et de niveau des vis pédiculaires, sans contrainte sur les éléments d'ancrage osseux, aussi bien en flexion qu'en arrachement. Il en résulte une simplification et une facilité de mise en place de l'instrumentation très appréciables pour les chirurgiens par rapport aux instrumentations antérieures connues. En effet, il suffit aux chirurgiens, pour ces ancrages intermédiaires, de faire subir les torsions voulues aux connecteurs 1 afin de les adapter à chaque anatomie locale. Une fois cette déformation plastique réalisée, les connecteurs 1 intermédiaires ne subissent plus d'autres déformations, car les faibles sollicitations à ces différents emplacements sont insuffisantes pour provoquer leur déformation ultérieure.

D'une manière générale, le dispositif d'ostéosynthèse visé par l'invention est muni d'au moins un connecteur (1) malléable et déformable plastiquement.

## Revendications

1. Dispositif d'ostéosynthèse rachidienne, comprenant au moins une tige vertébrale (2), des vis pédiculaires (3, 13), des connecteurs (1, 10) de liaison entre la tige et les vis, chaque connecteur comprenant deux parties (4, 6) respectivement traversées par la vis et par la tige, **caractérisé en ce que** les connecteurs (1;10) présentent chacun une lumière oblongue (5) s'étendant transversalement à la tige et adaptée pour être traversée par la vis associée, et au moins un connecteur (1) est réalisé en un matériau malléable, plastiquement déformable dans sa partie (4) traversée par la vis (3) sous l'effet du serrage d'un écrou (8) sur cette vis lors de sa mise en place, et **en ce que** ce connecteur est indéformable sous l'effet de moments de flexion transmis par le rachis in vivo et dont les valeurs sont inférieures à 4,5N.m. environ.

2. Dispositif selon la revendication 1, **caractérisé en ce que** le matériau malléable constituant le connecteur plastiquement déformable présente une capacité d'allongement d'au moins 20%.

3. Dispositif selon l'une des revendications 1 et 2, **caractérisé en ce que** les connecteurs intermédiaires (1) entre des connecteurs (10) fixés aux extrémités de la tige (2) sont réalisés en ledit matériau malléable, plastiquement déformable, les connecteurs d'extrémités (10) étant en un matériau indéformable.

4. Dispositif selon l'une des revendications 1 à 3, **caractérisé en ce que** le connecteur déformable (1) est en titane pur ou en acier inoxydable 316L recuit, ayant des caractéristiques de ductilité élevées.

5. Connecteur pour dispositif d'ostéosynthèse rachidienne selon l'une des revendications 1 à 4, **caractérisé en ce qu'**il est réalisé en un matériau malléable, plastiquement déformable dans sa partie (4) traversée par la vis (3) sous l'effet du serrage d'un écrou (8) sur la vis lors de sa mise en place, et ce connecteur est indéformable sous l'effet de moments de flexion transmis par le rachis in vivo et dont les valeurs sont inférieures à 4,5N.m. environ.

## Claims

1. Spinal osteosynthesis device, comprising at least one vertebral rod (2), pedicle screws (3, 13), and connectors (1, 10) linking the rod and the screws, each connector comprising two parts (4, 6) through which the screw and the rod pass respectively, **characterized in that** the connectors (1, 10) each have an oblong hole (5) extending transversely with respect to the rod and designed to be passed through by the associated screw, and at least one connector (1), is made of a malleable material plastically deformable in its part (4) passed through by the screw (3), under the effect of the tightening of a nut (8) on this screw during its placement, and **in that** this connector is non-deformable under the effect of bending moments which are transmitted by the spine *in vivo* and whose values are less than approximately 4.5 N.m.

2. Device according to Claim 1, **characterized in that** the malleable material constituting the plastically deformable connector has a capacity of elongation of at least 20%.

3. Device according to one of Claims 1 and 2, **characterized in that** the intermediate connectors (1) between connectors (10) fixed to the ends of the rod (2) are made of said malleable, plastically deformable material, the end connectors (10) being made of a non-deformable material.

4. Device according to one of Claims 1 to 3, **characterized in that** the deformable connector (1) is made of pure titanium or of annealed stainless steel 316L, having high ductility characteristics.

5. Connector for a spinal osteosynthesis device according to one of Claims 1 to 4, **characterized in that** it is made of a malleable material plastically deformable, in its part (4) passed through by the screw (3), under the effect of the tightening of a nut (8) on the screw during its placement, and this connector is non-deformable under the effect of bending moments which are transmitted by the spine *in vivo* and whose values are less than approximately 4.5 N.m.

## Patentansprüche

1. Vorrichtung zur Wirbelsäulenosteosynthese mit zumindest einer Wirbelstange (2), gestielten Schrauben (3, 13), Verbindungselementen (1, 10) zur Verbindung der Stange und der Schrauben, wobei jedes Verbindungselement zwei Teile (4, 6) umfasst, die von der Schraube beziehungsweise der Stange durchquert werden, **dadurch gekennzeichnet, dass** die Verbindungselemente (1, 10) jeweils eine längliche Öffnung (5) aufweisen, die sich transversal zur Stange erstreckt und dafür ausgelegt ist, von der zugehörigen Schraube durchquert zu werden, und dass zumindest ein Verbindungselement (1) aus einem streckbaren Material hergestellt ist, das in seinem von der Schraube (3) durchquerten Teil (4) unter der Wirkung des Festziehens einer Mutter (8) auf dieser Schraube bei ihrer Positionierung plastisch verformbar ist, und dass dieses Verbindungselement unter der Einwirkung von Biegemomenten, die von dem Rückgrat in vivo übertragen werden und deren Werte kleiner als etwa 4,5 Nm sind, unverformbar ist.

2. Vorrichtung nach Anspruch 1, **dadurch gekennzeichnet, dass** das streckbare Material, das das plastisch verformbare Verbindungselement bildet, eine Dehnfähigkeit von zumindest 20% besitzt.

3. Vorrichtung nach einem der Ansprüche 1 und 2, **dadurch gekennzeichnet, dass** die Zwischenverbindungselemente (1) zwischen an den Enden der Stange (2) befestigten Verbindungselementen (10) aus dem streckbaren, plastisch verformbaren Material hergestellt sind, während die Verbindungselemente der Enden (10) aus einem unverformbaren Material sind.

4. Vorrichtung nach einem der Ansprüche 1 bis 3, **dadurch gekennzeichnet, dass** das verformbare Verbindungselement (1) aus reinem Titan oder aus rostfreiem, geglühtem Stahl 316L ist, der Eigenschaften erhöhter Duktilität aufweist.

5. Verbindungselement für eine Vorrichtung zur Wirbelsäulenosteosynthese gemäß den Ansprüchen 1 bis 4, **dadurch gekennzeichnet, dass** es aus einem streckbaren Material hergestellt ist, das in seinem von der Schraube (3) durchquerten Teil (4) unter der Wirkung des Festziehens einer Mutter (8) auf der Schraube bei ihrer Positionierung plastisch verformbar ist, und dass dieses Verbindungselement unter der Einwirkung von Biegemomenten, die von dem Rückgrat in vivo übertragen werden und deren Werte kleiner als etwa 4,5 Nm sind, unverformbar ist.
